# EUROPEAN PATENT APPLICATION

(11) **EP 1 419 760 A1**
(43) Date of publication of application: **19.05.2004**
(21) Application number: 03024576.5
(22) Date of filing: 28.10.2003
(51) Int. Cl.: A61K 7/32

(54) **Antiperspirant compositions of enhanced efficacy containing strontium**

(30) Priority: 12.11.2002 US 292861
(71) Applicant: REHEIS, INC., Berkeley Heights New Jersey 07922 (US)
(72) Inventor: Li, Zijun, Westfield, New Jersey 07090 (US); Parekh, Jawahar C., Livingston, New Jersey 07039 (US)
(74) Representative: Hertz, Oliver, Dr.

(57) **Abstract**

Antiperspirant actives of aluminum and aluminum-zirconium of enhanced efficacy containing strontium and an amino acid that have a stable high HPLC Band III/II ratio are disclosed. The invention also discloses method for making the antiperspirant actives.

## Description

This invention relates to an antiperspirant solution having improved efficacy that contains strontium and amino acid. More particularly, this invention relates to an enhanced efficacy antiperspirant aqueous solution that has high HPLC Band III/II ratio at relatively higher active concentration and that high Band III/II ratio is stabilized upon aging.

### BACKGROUND OF THE INVENTION

Enhanced efficacy aluminum and aluminum-zirconium compositions, depending on the analytical procedures used, generally have Band III/II (or peak 4/3) area ratio of at least 0.5 - 0.9, with at least 70% aluminum contained in said bands. Such solutions, however, are unstable, i.e. revert back to their non-enhanced state, which means Band III/II peak area ratio falls below 0.3, particularly at concentration greater than 20% by weight. The aluminum and aluminum-zirconium salts of enhanced efficacy are only commercially available in powder form.

US 6,042,816 discloses a stable antiperspirant solution of enhanced efficacy containing calcium and an amino acid and methods of making the solution. By "stable" is meant that the HPLC Band III/II area ratio will remain 0.5 or higher, preferably at least 0.7, for at least one month at room temperature. It particularly stated in the patent, however, that "when similar solutions were tested with calcium replaced by magnesium, tin, zinc, barium and strontium, the 4/3 peak ratio was not stabilized."

It is surprisingly found by the present invention that aluminum and aluminum-zirconium antiperspirant solutions containing strontium and an amino acid demonstrate a HPLC chromatogram that has high Band III, and the area ratio of Band III/II remained above 0.7 for at least one month at room temperature.

### SUMMARY OF THE INVENTION

The antiperspirant solutions of the present invention have enhanced efficacy and stability and contain relatively higher concentration of antiperspirant actives due to the presence of strontium together with an amino acid. Such solution can be prepared both in aqueous and polyhydric alcohol solutions.

The composition of the present invention can be simply prepared by heating an aluminum or aluminum-zirconium antiperspirant solution with a strontium salt and an amino acid for a suitable period of time.

An alternative method involves reacting aluminum with an aqueous solution of aluminum salt of aluminum halide or aluminum nitrate containing a strontium salt and an amino acid, in the presence or absence of a zirconium complex.

### DETAILED DESCRIPTION OF THE INVENTION

The aluminum and aluminum-zirconium antiperspirant solutions of enhanced efficacy of the present invention contain 1) 5 to 35 weight percent and comprise those having the formula:

Al₂(OH)₆₋ₓ₁ Yₓ₁(R)p

wherein Y is Cl, Br, I and/or NO₃ and x₁ is greater than zero and less than or equal to six (i.e., 0<x₁≤6); and wherein "R" is an organic solvent having at least two carbon atoms and at least one hydroxy group and "p" has a value of from zero to 5; 2) an amino acid; and 3) a strontium salt. The antiperspirant contemplated include also the reaction products of those of the above formula and zirconium compounds of the formula

ZrO(OH)_{2-ab}X_{b}

wherein X is a member selected from the group consisting of halide, nitrate, pechlorate, carbonate or sulfate; b varies from 0.5 to 2; a is the valence of X; (2-ab) is greater than or equal to zero.

The preferred solutions exhibit a HPLC Band III to Band II area ratio of at least 0.7, which remain stable for at least a month at room temperature. At least 70%, preferably at least 80% of aluminum is contained in Bands II and III, as well as in IV. The solution consists of less than 10%, i.e. about 0 to about 10% of Al^{b}*type of species, which is believed to be less efficacious.
* Refers to aluminum species as measured using Ferron method by Hem and Roberson and Hem and Smith (Ref. "Antiperspirant and Deodorants", Carl Laden et al 1988, pg 141).

The amount of strontium in the solution should be from about 1 to about 6% by weight, preferably from about 2 to about 5%, and most preferably about 3 to 4%. Preferred strontium salt include strontium chloride, strontium bromide, strontium nitrate, strontium citrate, strontium formate, strontium lactate, strontium glycinate, strontium sulfate, strontium carbonate, and strontium hydroxide and the mixture thereof.

The composition of the present invention also contains an amino acid. Suitable amino acids useful herein have a number of amino groups that equals the number of carboxyl groups in the molecule, such as glycine. Other suitable amino acid compounds which can be used include aluminum, calcium, magnesium, sodium, alkaline and alkaline earth glycinates, zinc glycinate and the like, DL-valine, alaine argininne, L-proline, etc. and mixtures thereof. The preferred amino acids include glycine, alanine and valine, with glycine being the most preferred. The amount of amino acid used should be about 2% to about 8% by weight, preferably about 3% to about 7%, most preferably from about 4% to about 6%.

The degree of polymerization of aluminum complexes is determined by the high performance liquid chromatography (HPLC). The highest molecular weight Al species are eluted first, designated as Band I. Bands II and III designate intermediate molecular weight Al complexes. Band IV designates the lowest molecular weight Al complexes, including monomers and dimers. The relative area of one or more peaks is determined in order to characterize the distribution of polymeric species in the aluminum complexes formed. Desirably, the percent of Band III peak area of the composition of the invention is from about 35 to about 75%. The area ratio of Band III/II is greater than 0.7, with at least 70% Al species contained in Bands II, III and IV.

A Phenominex column (RP2) is used to obtain the HPLC chromatograph. A sample of a 2% by weight solution of Al or Al-Zr is filtered through a 0.45 micron filter and chromatographed within about 5 to about 10 minutes using a 0.01N nitric acid solution as the mobile phase.

The present invention provides methods of making a stable aluminum and aluminum-zirconium antiperspirant solution that contains strontium and amino acid having high Band III/II ratio and the ratio remains stable upon aging.

The invention will be further described in the following Examples. In the Examples, parts are by weight.

### Example 1

Basic aluminum chlorohydrate (200 parts) having an Al : Cl ratio of 1.9:1 was mixed with 41 parts of strontium chloride hexahydrate and 25 parts of glycine, to which 191 parts of water was added. The solution was heated to reflux for two hours using a reflux condenser.

### Example 2

This example was made similar to Example 1 except that the concentration of the anhydrous solid was increased.

### Example 3

In this example polyhydric alcohol, i.e. propylene glycol (PG) was used (5.45%) together with water.

### Examples 4 and 5

In these examples the aluminum to zirconium atomic ratio was varied from 5.63 to 9.25.
The results of Examples 1 - 5 are set fourth below in the Table I.

**TABLE I**

| | %Al | %Zr | %Cl | %glycine | %Sr | %A.S. | %Al^{b} | Al/Zr |
|---|---|---|---|---|---|---|---|---|
| Example 1 | 5.15 | - | 6.04 | 5.46 | 2.96 | 18.0 | 2.0 | |
| Example 2 | 7.93 | - | 7.99 | 5.84 | 3.02 | 27.1 | 7.2 | |
| Example 3 | 7.85 | - | 8.06 | 5.82 | 3.01 | 26.9 | 0.0 | |
| Example 4 | 4.69 | 2.87 | 7.78 | 4.84 | 2.84 | 22.6 | 4.4 | 5.63 |
| Example 5 | 7.01 | 2.61 | 9.03 | 5.48 | 2.82 | 29.5 | 5.2 | 9.25 |

Shown in Table II are the stability data of HPLC Band III/II peak area ratio of solutions of Examples 1-5 for up to 6 months.

**TABLE II**

| | Initial III/II | 1 MO III/II | 2 MO III/II | 3 MO III/II | 4 MO III/II | 5 MO III/II | 6 MO III/II |
|---|---|---|---|---|---|---|---|
| Example 1 | 2.6 | 2.9 | 2.5 | 2.7 | 2.9 | 3.1 | 2.5 |
| Example 2 | 1.5 | 1.6 | 2 | 2 | 2 | 2.2 | 2.3 |
| Example 3 | 1.5 | 1.6 | 2.1 | 1.8 | 1.7 | 1.9 | 2.0 |
| Example 4 | 1.8 | 1.9 | 2.1 | 1.9 | 1.9 | 1.9 | 2.1 |
| Example 5 | 1.6 | 1.5 | 1.8 | 1.8 | 1.8 | 1.6 | 1.5 |

### Example 6

52 parts of 32° Baume aluminum chloride, 89 parts of zirconium oxychloride, 20 parts of glycine, 30 parts of strontium chloride hexahydrate, 23 parts of aluminum and 285 parts of propylene glycol were mixed and heated to about 120°C. An almost clear solution was obtained after 7.5 hrs, which was filtered to give a clear light yellowish solution. Chemical analysis: 5.42%Al, 4.7%Zr, 7.8%Cl, 2.87%glycine, 59.87%PG, and 1.92%Sr with 7.1%Al^{b} by Ferron analysis. Initial Band III/II was 7.2 and was 7.1 after 6 MO.

Although the present invention has been described in terms of specific embodiments, the invention is not meant to be so limited. Various changes in the ingredients and their amounts can be substituted while still obtaining the benefits of the invention. Thus the invention is only meant to be limited by the scope of the appended claims.

## Claims

1. A stable enhanced efficacy antiperspirant composition comprising:
a) about 1 to about 6% by weight of strontium, and
b) from about 2 to 8% by weight of an amino acid having HPLC Band III/II peak area ratio of no less than 0.7 and having the formula
Al₂(OH)₆₋ₓ₁ Yₓ₁(R)p
wherein Y is Cl, Br, I and/or NO₃ and x₁ is greater than zero and less than or equal to six (i.e., 0<x1≤ 6); wherein "R" is an organic solvent having at least two carbon atoms and at least one hydroxy group and "p" has a value of from zero to 5.

2. The composition according to claim 1 which includes a zirconium salt of the formula
ZrO(OH)_{2-ab}X_{b}
wherein X is a member selected from the group consisting of halide, nitrate, perchlorate, carbonate or sulfate; b varies from 0.5 to 2; a is the valence of X; (2-ab) is greater than or equal to zero.

3. The composition of claim 1 or 2 wherein the antiperspirant active materials are covered by FDA OTC Tentative Final Monograph as Category I.

4. The composition according to claim 1 or 2 wherein the concentration of the antiperspirant active comprises from about 5 to about 35 weight percent.

5. The composition according to claim 1 compromising a stable HPLC Band III/II of at least 0.7.

6. The composition according to claim 1 wherein the strontium salt is selected from the group consisting of strontium chloride, strontium bromide, strontium nitrate, strontium citrate, strontium formate, strontium lactate, strontium glycinate, strontium sulfate, strontium carbonate, strontium hydroxide and the mixtures thereof.

7. The composition according to claim 2 wherein the aluminum: zirconium atomic ratio is about from 1 : 10 to 10 : 1 and the metal to anion ratio is from about 0.9 : 1 to 2.1:1.

8. The composition according to claim 1 wherein the organic solvent is a polyhydric alcohol having at least two carbon atoms and least two hydroxy groups.

9. The composition according to claim 1 or 2 that is dried to a solid product.

10. A method of forming a stable enhanced efficacy antiperspirant solution having HPLC Band III/II peak area ratio of at least 0.7 comprising:
a) heating an admixture of (i) at least 10 weight percent and not more than 35 weight percent of a solution of an antiperspirant active having the formula:
Al₂(OH)₆₋ₓ₁ Yx₁(R)p
wherein Y is Cl, Br, I and/or NO₃ and x₁ is greater than zero and less than or equal to six (i.e., 0<x1≤6); and wherein "R" is an organic solvent having at least two carbon atoms and at least one hydroxy group and "p" has a value of from zero to 5; (ii) an amino acid, and (iii) a strontium salt; and
b) maintaining the application of heat until a solution of enhanced efficacy is formed.

11. The method according to claim 10 wherein the concentration of strontium in solution is from 1 to 6% by weight.

12. The method according to claim 10 wherein the concentration of amino acid is from 2 to 8% by weight.

13. The method according to claim 10 comprising adding to the admixture a zirconium salt of the formula
ZrO(OH)_{2-ab}X_{b}
wherein X is a member selected from the group consisting of halide, nitrate, perchlorate, carbonate or sulfate; b varies from 0.5 to 2; a is the valence of X; (2-ab) is greater than or equal to zero.

14. The method according to claim 10 wherein the heating of the admixture is maintained at reflux from 1 to 4 hrs.

15. The method according to claim 10 or 13 containing a polyhydric alcohol having at least two carbon atoms and at least two hydroxy groups.

16. The method according to claim 10 or 13 including the step of drying the solution to a solid form.

17. A method of forming an aluminum antiperspirant active having a HPLC Band III/II ratio of at least 0.7 and having from about 1 to 6% by weight of strontium and about 2 to 8% by weight of amino acid which comprises reacting an admixture of aluminum metal with an aqueous solution of an aluminum salt of a halide, nitrate or sulfate in the presence of an amino acid and strontium salt.

18. The method according to claim 17 which includes adding a zirconium salt of the formula
ZrO(OH)_{2-ab}X_{b}
wherein X is a member selected from the group consisting of halide, nitrate, perchlorate, carbonate or sulfate; b varies from 0.5 to 2; a is the valence of X; (2-ab) is greater than or equal to zero.

19. The method according to claim 10, 13, 17 or 18 wherein the concentration of the antiperspirant active is from about 5 to about 35 weight percent.

20. The method according to claim 10 or 17 wherein the strontium salt is selected from the group consisting of strontium chloride; strontium bromide, strontium nitrate, strontium citrate, strontium formate, strontium lactate, strontium glycinate, strontium sulfate, strontium carbonate, strontium hydroxide and the mixture thereof.

21. The method according to claim 13 or 18 wherein the aluminum : zirconium atomic ratio is from 1 : 10 to 10 : 1 and having a metal to anion atomic ratio of about 9.1:1 to 2.1:1.

22. The method according to claim 17 or 18 wherein the aqueous solution contains a polyhydric alcohol having at least two carbon atoms and at least two hydroxy groups.

23. The composition according to claim 17 or 18 that is dried to a solid product.
